# EUROPEAN PATENT APPLICATION

(11) **EP 0 700 670 A1**
(43) Date of publication of application: **13.03.1996**
(21) Application number: 95305443.4
(22) Date of filing: 03.08.1995
(51) Int. Cl.: A61F 2/36, A61B 17/16

(54) **Femoral component for hip joint replacement**

(30) Priority: 11.08.1994 GB 9416216
(71) Applicant: Crawshaw, Charles, Redmarley D'Abitot, Gloucestershire (GB); Curtis, Mark J., Kingston-on-Thames, Surrey KT2 7BW (GB)
(72) Inventor: Crawshaw, Charles, Redmarley D'Abitot, Gloucestershire (GB); Curtis, Mark J., Kingston-on-Thames, Surrey KT2 7BW (GB)
(74) Representative: Moir, Michael Christopher

(57) **Abstract**

A femoral component for use in a cementless hip joint replacement has an axially tapering distal portion (10) and an axially tapering proximal portion (12), said proximal portion (12) tapering with an included angle of taper of at least 8° in both the medial-lateral and anterior-posterior directions, a medially-extending stem (14) diverging from the proximal portion (12) for supporting a prosthetic bearing surface (16), the lateral surface (22) of the proximal portion (12) being convex when viewed in cross-section orthogonally of the longitudinal axis of the component, the cross-sectional width of the lateral convex surface (22) of the proximal portion (12) being substantially greater than the cross-sectional width of the medial surface thereof, whereby the proximal portion (12) provides the primary means of engagement with a prepared conforming intra-femoral seat to transfer in-service loads to said femur, the distal portion (10) being adapted to fit within the femoral intramedullary canal to stabilize the component relative to the femur substantially without transferring in-service loads thereto.

## Description

This invention relates to a femoral component for a hip joint replacement, to a system comprising the combination of such a component and tooling for preparation of the femur for reception thereof, and to a method of replacing the femoral component of a hip joint.

Femoral implants for hip joint replacements hitherto have been shaped at their proximal (upper) portions to fit within a seat prepared within the upper part of the femur with minimum removal of healthy bone, it being believed that as much as possible of the femoral bone should be retained. The distal or lower portion of the implant is received in the medullary canal of the femur after suitable preparation thereof. Problems which arise in these known implants are those of "stress transfer" and "stress shielding", in which loads between the femur and the implant are either concentrated other than where intended in the proximal portion, or indeed are instead transferred via the distal portion to the femur. The result is that parts of the femur are overstressed, leading to pain and abnormal bone growth, or are understressed, leading to wasting of the unstressed bone. In extreme cases of overstressing, eg. if a substantial load is transferred via the tip of the distal portion, fracture of the femur and/or loosening or failure of the component may occur.

The present invention seeks to eliminate or at least reduce these problems by specifying a component in which the proximal portion is fashioned to require removal of a greater quantity of bone than in previous practice in order to provide a conical or otherwise relatively sharply-tapering seat in cortical femoral bone through which loads are transferred.

Thus in one aspect the invention provides a femoral component for use in a cementless hip joint replacement, the component having an axially tapering distal portion and an axially tapering proximal portion, said proximal portion tapering in both the medial-lateral and anterior-posterior directions, a medially-extending stem diverging from the proximal portion for supporting a prosthetic bearing surface, the medial and lateral surfaces of the distal and proximal portions being convex when viewed in cross-section orthogonally of the longitudinal axis of the component, the cross-sectional width of the lateral convex surface of the proximal portion being substantially greater than the cross-sectional width of the medial convex surface thereof, the dimensions and taper of the proximal portion being adapted to engage a conforming intra-femoral seat prepared in cortical bone whereby to transfer in-service loads to said femur, the distal portion being adapted to fit within the femoral intramedullary canal to stabilize the component relative to the femur substantially without transferring in-service loads thereto.

In a further aspect the invention provides a femoral component for use in a cementless hip joint replacement, the component having a distal portion and a proximal portion comprising a stem diverging therefrom on its medial side for supporting a prosthetic bearing surface, the lateral side of the proximal portion being of substantially conical form adapted to engage a conforming intra-femoral seat prepared in cortical bone to provide the primary means of transferring in-service loads to the femur, the diverging stem being also adapted to engage said conforming seat to prevent rotation of the component therein, the distal portion being adapted to fit within the femoral intramedullary canal to stabilize the component relative to the femur substantially without transferring in-service loads thereto.

The invention also provides a method of replacing the femoral component of a hip joint, comprising preparing a femoral medullary canal to receive a distal portion of a femoral implant, preparing a seat in femoral cortical bone to receive a proximal portion of the implant, the femoral seat and the proximal portion comprising conforming profiles of substantially conical form having an included angle of at least 8 degrees and the medullary canal being prepared so as to be oversize relative to the distal portion whereby the femoral seat receives primary in-service loads from the proximal portion of the implant and the distal portion fits within the medullary canal to stabilise the implant relative to the femur substantially without transferring in-service loads thereto.

The term conical is used herein in a broad sense to include diverging forms having a generally curvilinear but not necessarily circular cross-section orthogonally to a longitudinal axis.

The proximal portion may have an included angle of at least 8 and preferably between 8 and 12 degrees, for example 10 degrees.

The included angle preferably is at least 8 degrees in both the anterior-posterior and medial-lateral planes.

In another aspect the invention provides a femoral component for use in a cementless hip joint replacement, the component having an axially tapering distal portion and an axially tapering proximal portion, said proximal portion tapering with an included angle of taper of at least 8° in both the medial-lateral and anterior-posterior directions, a medially-extending stem diverging from the proximal portion for supporting a prosthetic bearing surface, the lateral surface of the proximal portion being convex when viewed in cross-section orthogonally of the longitudinal axis of the component, the cross-sectional width of the lateral convex surface of the proximal portion being substantially greater than the cross-sectional width of the medial surface thereof, whereby the proximal portion provides the primary means of engagement with a prepared conforming intra-femoral seat to transfer in-service loads to said femur, the distal portion being adapted to fit within the femoral intramedullary canal to stabilize the component relative to the femur substantially without transferring in-service loads thereto.

The medial and lateral surfaces may be joined by surfaces which are rectilinear when viewed in said cross-section.

Preferably, the medial edge of the proximal portion viewed in the medial-lateral plane is arcuate.

The said medial surface may be convex and preferably of constant radius.

The diverging stem may be blended to the lateral side of the proximal portion by surfaces which are substantially tangential to the conical form.

The invention also provides a system for replacing the femoral part of a hip joint, comprising a femoral component as set forth above, and a rasp for defining a seat to be cut into cortical femoral bone, the dimensions of the seat-defining portions of the rasp being such as to produce a seat conforming to the femoral component.

The system may further comprise a rotatable tapered reamer for initial removal of at least cancellous femoral bone to permit insertion of the rasp into the femur.

Preferably the reamer has the same angle of taper as the proximal portion of the component.

Preferably a portion of the rasp corresponding to a lower part of the proximal portion of the component is oversize relative thereto, and a portion of the rasp corresponding to an upper part of the proximal portion is undersize relative thereto.

The angles of taper of the rasp may conform to those of the proximal portion.

The invention will now be described merely by way of example with reference to the accompanying drawings, wherein:
Figure 1 is a perspective view of a femoral component according to the invention;
Figure 2 shows the component in more detail from lateral and anterior/posterior viewpoints, and in section;
Figure 3 shows a rasp forming part of a system according to the invention;
Figure 4 shows a reamer also forming part of the system.

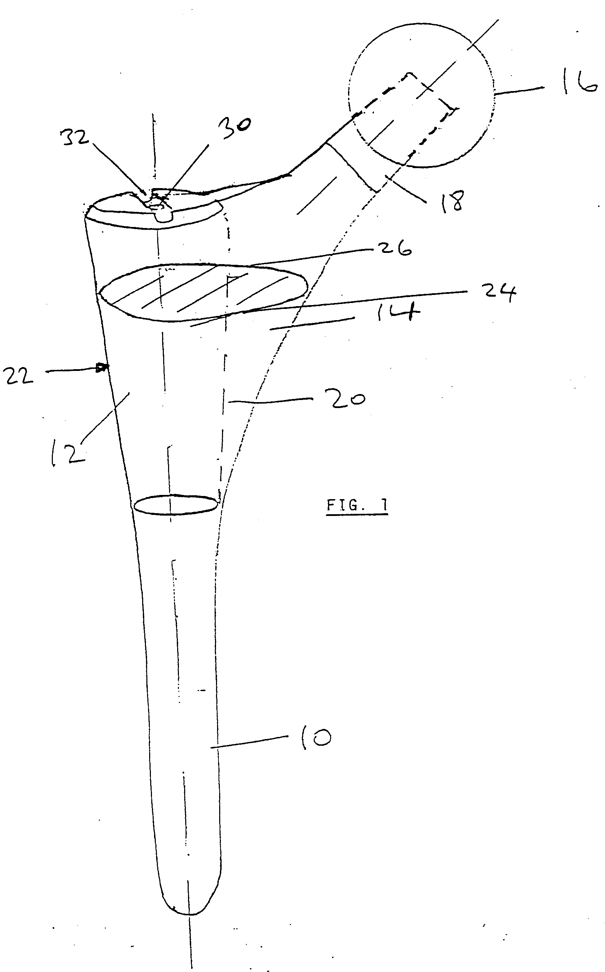

Referring to Figure 1, a femoral implant comprises a lower or distal portion 10 and an upper or proximal portion 12. A stem 14 diverges from the proximal portion to support a polished metal (eg. stainless steel or chrome cobalt) sphere 16 which in use is received in a prosthetic acetabular bearing cup to provide a hip joint replacement, as known per se in the art.

The sphere 16 is supported on the end part of the stem by means of conforming right conical male and female surfaces, also as known per se. The end part 18 of the stem is detachable to permit revision of the joint should the patient require further attention at a later date.

The component is manufactured in titanium alloy or a CoCrMo alloy, as known in the art. The distal portion is polished because it is not required that bone should grow onto and adhere to this part of the component. The proximal portion however has a shot-blasted surface to promote secure keying of the component into a prepared femoral seat, and subsequent adhesion of the bone thereto. Alternatively or in addition, it may have a plasma or sprayed hydroxyapatite coating for the same purpose.

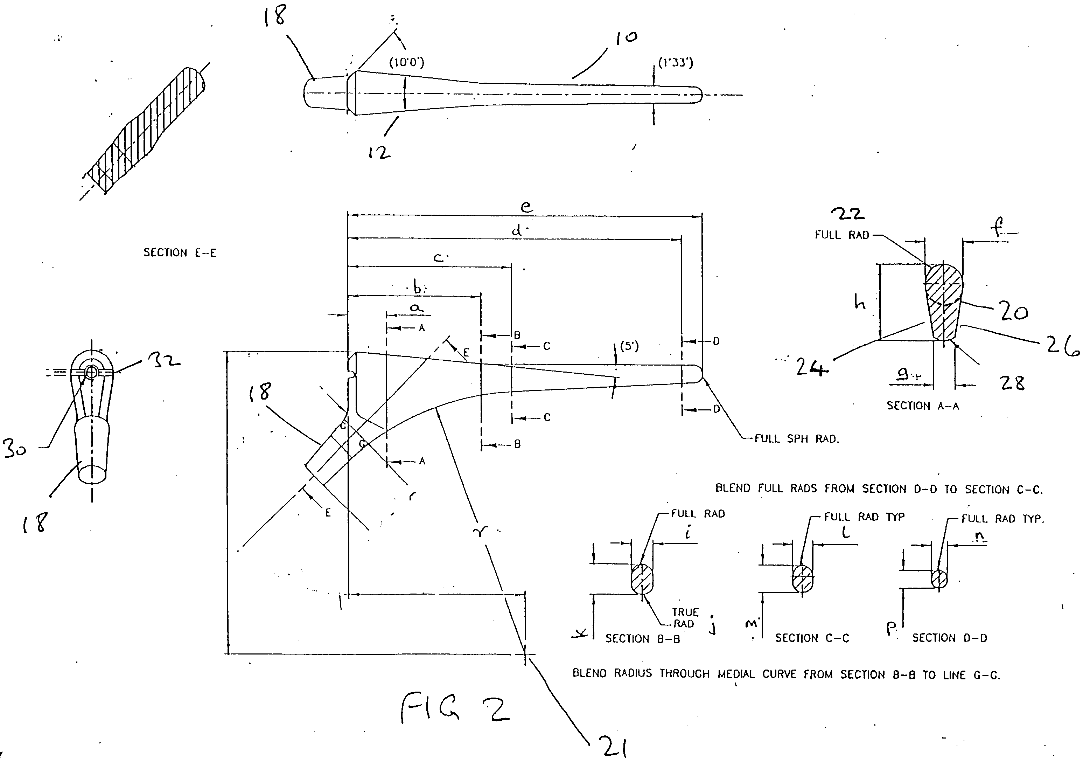

As best seen in Figure 2, the distal portion 10 tapers from its junction with the proximal portion 12 at approximately section C to its tip, changing in section from oval to circular. The included angle of taper in the anterior-posterior (A-P) plane is approximately 1.5 to 1.6 degrees, and about 3 degrees in the medial-lateral (M-L) plane.

The tip of the distal portion is relieved so as to have a spherical surface, so avoiding any sharp change in section which could locally raise the stress in the femur.

The proximal portion 12 is in the form of a cone, the stem 14 springing from one side of the cone and being blended to the conical surface. The position of the notional conical surface beneath the stem is shown dotted at 20 in Figures 1 and 2. In this example, it is circular in section. The conical surface of the proximal portion diverges significantly more rapidly than the distal portion. That is to say, the proximal portion has a considerably greater included angle or conical angle. Thus, in the preferred form as shown in Figure 2, the included angle is 10 degrees in both the M-L and A-P planes.

The medial edge of the stem 14 is curved, in this example as an arc struck from a centre 21 chosen so as to blend the curve to the proximal portion at approximately section B-B, Figure 2.

The width (dimension g, Figure 2) of the stem 14 at its medial side remains constant from section B-B to section A-A, whilst the width of the proximal portion at its lateral side increases due to the conical form. As shown typically at section A-A the half-round conical form 22 on the lateral side of the A-P mid-plane is blended to the medial surface by substantially planar side surfaces 24,26 which are approximately tangential to the semi-circular section 22. The sectional form 28 of the medial surface, whilst convex, is not rounded to the extent of blending with the surfaces 24,26 as tangents. The sectional form 28 may be further flattened so as to be substantially planar in its medially-facing portion rather than curved, but is still convex in the sense of having no concavity or re-entrant angles.

The proximal portion is provided with a threaded bore 30 and slot 32 to permit withdrawal or rotational adjustment of the component during the surgical procedure.

The conically-expanding proximal portion and the diverging stem provides the component with considerably greater cross-sectional dimensions (particularly in the A-P plane) than in prior art prosthetic implants, and the large conical angle ensures that the component can securely engage a prepared femoral seat with less risk that the implant will be driven further into the femur by in-service loads as sometimes happens with prior art components having non-diverging or only slightly diverging proximal portions. In other words the broad conical form provides for accurate axial registration of the component in the femur and at the same time ensures that loads are transferred via the conical surface to the femoral seat, and not elsewhere.

The lower part of the stem 14 is also received within the prepared femoral seat, and locates the component against rotation about its longitudinal axis. The surfaces 24,26 also diverge in the A-P plane, being blended to the lateral side 22 of the proximal portion 12 and thus also present surfaces which contribute to the transfer of in-service loads to conforming surfaces of the femoral seat.

To accommodate the component 10 in the femur, it is necessary to remove significantly more bone that in previous procedures. Indeed, the femoral seat is largely cut in cortical bone which hitherto would have been left untouched. However, the enlarged bearing area and the greater conical angle, which permits loads to be transferred at lower hoop stress levels within the surrounding femoral bone, provide a secure implantation of the femoral component which is less likely to suffer stress shielding or stress transfer problems.

The following table gives typical dimensions of the component, in three sizes intended for small, medium, and large-boned individuals. These dimensions are illustrative and are not actual working dimensions.

| **Approximate Dimensions (mm)** | **Size 2 Small** | **Size 3 Medium** | **Size 4 Large** |
|---|---|---|---|
| a | 17 | 18.5 | 20 |
| b | 58 | 64 | 70 |
| c | 72 | 79 | 87 |
| d | 155 | 161 | 167 |
| e | 165 | 171 | 177 |
| f | 17 | 18.5 | 20.5 |
| g | 9 | 10.5 | 12 |
| h | 33 | 36 | 40 |
| i | 9 | 10.5 | 12 |
| j | 5 | 5 | 6.5 |
| k | 13 | 14.5 | 16 |
| l | 9 | 10 | 11 |
| m | 11.5 | 13 | 14 |
| n | 7 | 8 | 9 |
| p | 7.5 | 8.5 | 10 |
| r | 114 | 125 | 138 |

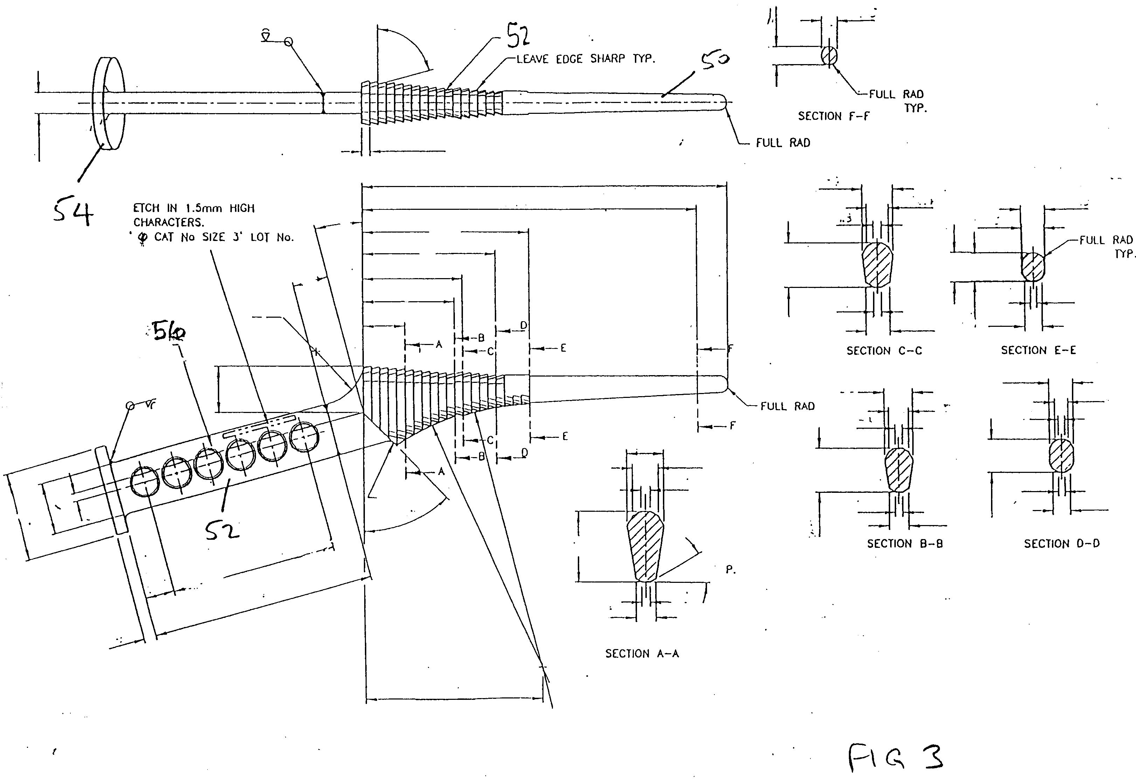

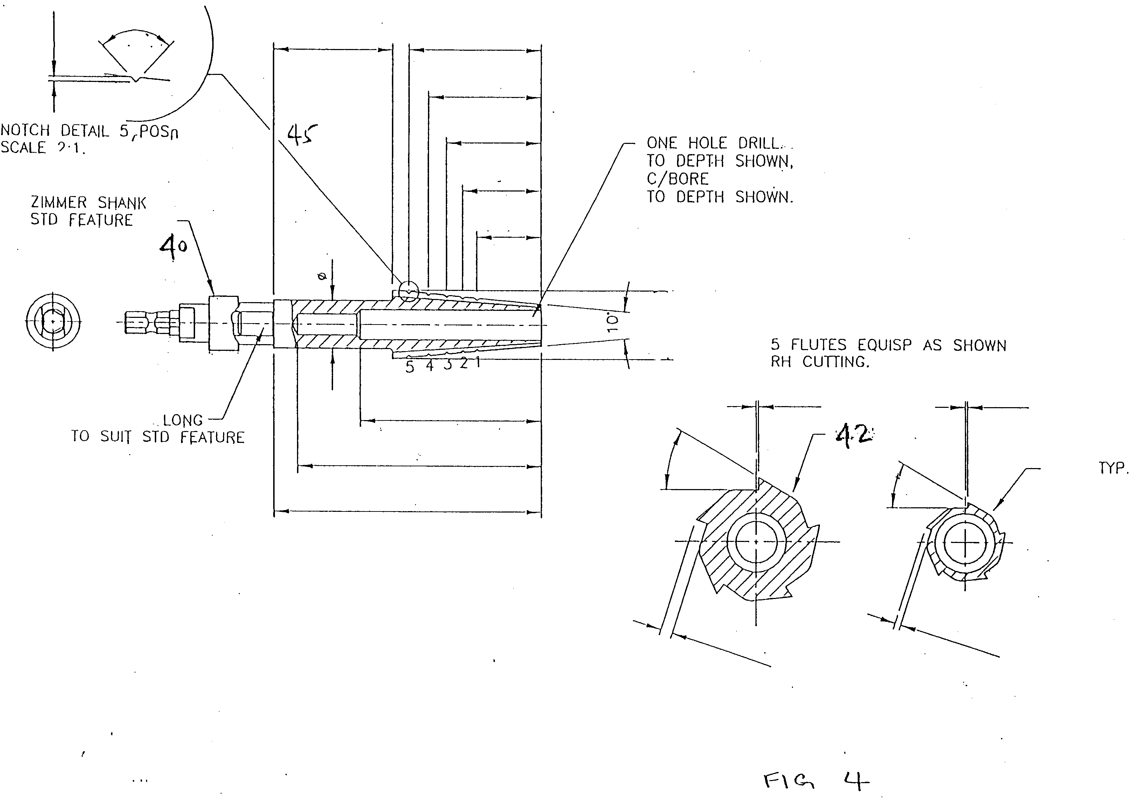

Figures 3 and 4 show respectively a rasp and a reamer for preparing the femoral seat. Dealing first with the reamer, the tool has a conventional shank 40 and five tapering flutes or cutting surfaces 42 with chip-breaking notches 44 at intervals. The reamer is distinguished from prior art reamers in that the angle of taper of the flutes is greater than hitherto and matches that of the proximal portion of the component 10, in this instance 10 degrees.

The reamer is employed to cut a 10° right conical seat in the proximal part of the femur, which is then modified by the rasp of Figure 4. The rasp has a tail portion 50 having the same dimensions between sections F-F and E-E as the distal portion 10 of the prosthetic component to which it corresponds (there is a rasp for each size of component) and a series of broach-like cutting teeth 52. A conventional handle 54 and striking head are provided, the handle having holes 56 to enable the rasp to be engaged and withdrawn from the femur after use.

Between sections E-E and C-C the rasp is larger than the corresponding parts of the implant, by approximately 0.5mm. Between section B-B and the last tooth the rasp is approximately 0.5mm smaller than the implant. The rasp thereby cuts a seat in the femoral bone which is oversize compared to the lower part of the proximal portion 12 but undersize compared to the upper part thereof.

When the implant is inserted in the femur, engagement with the femoral seat occurs in the upper part thereof rather than in the lower part where the femur is narrower. The distal portion is received in the medullary canal without transferring loads thereto, merely acting to stabilise the implant during insertion and bedding-down in the femoral seat. To achieve this the medullary canal is first enlarged by a suitably profiled rasp or broach so as to be slightly oversize relative to the distal portion of the implant.

Save for the foregoing, the preparation of the femur and the insertion of the implant follow established surgical techniques, and so will not be further discussed.

Each feature disclosed in this specification (which term includes the claims) and/or shown in the drawings may be incorporated in the invention independently of other disclosed and/or illustrated features.

The appended abstract as filed herewith is included in the specification by reference.

## Claims

1. A femoral component for use in a cementless hip joint replacement, the component having an axially tapering distal portion and an axially tapering proximal portion, said proximal portion tapering in both the medial-lateral and anterior-posterior directions, a medially-extending stem diverging from the proximal portion for supporting a prosthetic bearing surface, the medial and lateral surfaces of the distal and proximal portions being convex when viewed in cross-section orthogonally of the longitudinal axis of the component, the cross-sectional width of the lateral convex surface of the proximal portion being substantially greater than the cross-sectional width of the medial convex surface thereof, the dimensions and taper of the proximal portion being adapted to engage a conforming intra-femoral seat prepared in cortical bone whereby to transfer in-service loads to said femur, the distal portion being adapted to fit within the femoral intramedullary canal to stabilize the component relative to the femur substantially without transferring in-service loads thereto.

2. A component as claimed in Claim 1, wherein the proximal portion has an included angle of taper of at least 8 degrees.

3. A femoral component for use in a cementless hip joint replacement, the component having an axially tapering distal portion and an axially tapering proximal portion, said proximal portion tapering with an included angle of taper of at least 8° in both the medial-lateral and anterior-posterior directions, a medially-extending stem diverging from the proximal portion for supporting a prosthetic bearing surface, the lateral surface of the proximal portion being convex when viewed in cross-section orthogonally of the longitudinal axis of the component, the cross-sectional width of the lateral convex surface of the proximal portion being substantially greater than the cross-sectional width of the medial surface thereof, whereby the proximal portion provides the primary means of engagement with a prepared conforming intra-femoral seat to transfer in-service loads to said femur, the distal portion being adapted to fit within the femoral intramedullary canal to stabilize the component relative to the femur substantially without transferring in-service loads thereto.

4. A component as claimed in Claim 3, wherein the said medial surface is convex and of constant radius.

5. A component as claimed in Claim 2, 3 or 4, wherein the included angle of taper is at least 8 degrees in both the anterior-posterior and medial-lateral planes.

6. A component as claimed in any preceding claim, wherein the medial and lateral surfaces are joined by surfaces which are rectilinear when viewed in said cross-section.

7. A component as claimed in any preceding claim, wherein the medial edge of the proximal portion viewed in the medial-lateral plane is arcuate.

8. A femoral component for use in a cementless hip joint replacement, the component having a distal portion and a proximal portion comprising a stem diverging therefrom on its medial side for supporting a prosthetic bearing surface, the lateral side of the proximal portion being of substantially conical form adapted to engage a conforming intra-femoral seat prepared in cortical bone to provide the primary means of transferring in-service loads to the femur, the diverging stem being also adapted to engage said conforming seat to prevent rotation of the component therein, the distal portion being adapted to fit within the femoral intramedullary canal to stabilize the component relative to the femur substantially without transferring in-service loads thereto.

9. A femoral component as claimed in Claim 8, wherein the included angle of the conical form is at least 8 degrees.

10. A femoral component as claimed in Claim 8 or Claim 9, wherein the diverging stem is blended to the lateral side of the proximal portion by surfaces which are substantially tangential to the conical form.

11. A system for replacing the femoral part of a hip joint, comprising a femoral component as claimed in any preceding claim, and a rasp for defining a seat to be cut into cortical femoral bone, the dimensions of the seat-defining portions of the rasp being such as to produce a seat conforming to the femoral component.

12. A system as claimed in Claim 11, further comprising a rotatable tapered reamer for initial removal of at least cancellous femoral bone to permit insertion of the rasp into the femur.

13. A system as claimed in Claim 12, wherein the reamer has the same angle of taper as the proximal portion of the component.

14. A system as claimed in Claim 11, 12 or 13, wherein a portion of the rasp corresponding to a lower part of the proximal portion of the component is oversize relative thereto, and a portion of the rasp corresponding to an upper part of the proximal portion is undersize relative thereto.

15. A system as claimed in any of Claims 11 to 14, wherein the angles of taper of the rasp are equal to those of the proximal portion of the component.
